# EUROPEAN PATENT SPECIFICATION

(11) **EP 1 625 233 B1**
(45) Date of publication and mention of the grant of the patent: **21.11.2007**
(21) Application number: 04752269.3
(22) Date of filing: 14.05.2004
(51) Int. Cl.: C12Q 1/68, G01N 33/543

(54) **REDUCING MICROARRAY VARIATION WITH INTERNAL REFERENCE SPOTS**
REDUZIERUNG VON MIKROARRAY SCHWANKUNGEN DURCH INTERNE REFERENZPOSITIONEN
REDUCTION DE LA VARIATION D'UNE ANALYSE PAR MICRO-RESEAU AU MOYEN DE TACHES DE REFERENCE INTERNES

(30) Priority: 16.05.2003 US 439811
(43) Date of publication of application: 15.02.2006
(73) Proprietor: Beckman Coulter, Inc., Fullerton, CA 92834-3100 (US)
(72) Inventor: REDDY, Parameswara, Meda, Brea, California 92821 (US); BRILLHART, Kurt, Mission Viejo, California 92691-3438 (US); FAROOQUI, Firdous, Brea, California 92821 (US); KEYS, Daniel, A., Irvine, California 92604 (US)
(74) Representative: Müller-Boré & Partner Patentanwälte
(86) International application number: PCT/US2004/015209
(87) International publication number: WO 2004/104230

(56) References cited:
- EP-A- 1 132 136
- EP-A- 1 190 762
- EP-A- 1 203 945
- WO-A-00/73504
- WO-A-02/18655
- WO-A-02/090516
- WO-A-03/025580
- WO-A-03/054551

## Description

### BACKGROUND

The following description provides a summary of information relevant to the present invention and is not a concession that any of the information provided or publications referenced herein is prior art to the presently claimed invention.

A microarray is an array or pattern of discrete capture agents deposited as small spots (about 1mm or less) onto a suitable solid support. An example of a suitable solid support for the microarray, is a polyproplylene plate or other plate having a plurality of test areas onto which an array of spots can be deposited in the test areas. Reagents and a volume of sample are added to the test areas to detect target ligands.

An assay using a microarray can be performed a number of ways including the well-known sandwich technique. In the sandwich technique, target ligands, target ligand detectors, and capture ligands are used in the assay. "Target ligands" and "anti-ligands" are antigens, antibodies, binding proteins, haptens, hormone receptors, and other biological molecules that can form complexes by binding to other molecules. A capture ligand is an anti-ligand that binds to the target ligand. The "target ligand detector" is a labeled anti-ligand that also binds to the target ligand or target ligand-capture ligand complex.

In the sandwich assay the capture ligand binds to at least one site on a target ligand to form a first complex, thereby capturing or linking to the target ligand. A target ligand detector also binds to the target ligand to form a second complex. The labeled constituent in the sandwich of the complex can indicate the presence of a target ligand at a particular location in a microarray.

Microarrays are useful for detecting the presence of one or more target ligands using small volumes of sample. However, microarrays generally determine whether a target ligand is present, and do not quantify the target ligand. Quantitation of each target ligand in a microarray can be extremely useful or vital for many applications, and would improve and extend the usefulness of microarrays.

Precise quantification of target ligands in a microarray format is complex; variable conditions at any stage of the assay procedure can affect precision. These variations can be significant and decrease precision. Variable conditions can occur at one or more stage of the assay procedure from: 1) depositing capture agents as spots onto the microarray, 2) the sample and reagents onto the microarray, 3) incubating the sample and reagents, and/or 4) during the detection and measurement of the signals corresponding to each target ligand. The process of adding the sample or capture agents in the microarray can result in depositing variable amounts. If a visual label is used to detect a target ligand, then dust, uneven lighting, focusing problems, and/or instrument problems can create variability in the detection and measurement of the intensity of the label. The temperature, humidity, incubation period, and shaking are other variables that can occur during the assay using the microarray. Variation can also occur between different test areas within the array. All of these factors can affect the performance and precision of the microarray in detection and measurement of target ligands. There is a need for a microarray assay method that considers and adjusts for variables in detecting and measuring target ligands.

European Patent application EP 1190762 A2 in this respect discloses a method for displaying results of hybridization experiments using a biochip, wherein a plurality of control spots spotted in each of a plurality of sections defined on a biochip is measured. The measured data are plotted on a graph for each section, and all of the graphs are simultaneously displayed on a single screen in the same arrangement as that of the sections on the biochip. By simultaneously displaying all of the graphs on a single screen, it is possible to skim the whole biochip to find experimental errors. Also, experimental errors can be quantified with respect to the dispersion of control data on the basis of the linearity of the data points and slope angles defined for each data points on a graph.

Detecting and quantifying the intensity of the signal corresponding to each label directly or indirectly attached to each target ligand is further complicated because of the small size of the spots. Precise identification of the position of each capture site within each test area and the corresponding identity of each target ligand in the labeled complex is vital. Microarrays, typically lack internal markers or reference points that enable the user to easily and quickly determine the spot corresponding to each target ligand being tested in the microarray. The lack of internal reference points results in a microarray where a user has to engage in time consuming procedures to orient or align the spots corresponding to the target ligand captured in the microarray. Depending on the number of different target ligands that are being subjected to testing in the microarray, this can delay analysis of the assay results.

Accordingly, a need exists for an assay method for a microarray that: is quantitative; increases the precision of the microarray assay; facilitates orientation or alignment of the microarray to ensure accurate detection of spot in each arrays; and quantifies the captured target ligands without expensive and time consuming procedures.

### SUMMARY

The present invention satisfies that need. The present invention provides a system that uses internal reference spots to increase the precision and reduce microarray variation. The present invention's system includes microassay devices, assay methods, and methods of forming microassays.

A microarray device for determining the quantity of a plurality of target ligands in a sample, according to the present invention, comprises a solid support having a plurality of test areas; a plurality of different target capture ligands immobilized onto the test areas; and at least one reference capture ligand immobilized onto the test areas, the reference capture ligand being selected to capture a reference ligand not normally present in the sample.

Another microarray device for determining the quantity of a plurality of target ligands in a sample, according to the present invention, comprises a solid support having a plurality of test areas with substantially the same geometry on the solid support; a plurality of different target capture ligands immobilized onto the test areas; and at least one reference capture ligand immobilized onto the test areas, the reference capture ligand being selected to capture a reference ligand not normally present in the sample, the reference capture ligands being in substantially the same location in each test area.

Embodiments of the above microassay devices include devices that have one or more of the following: at least one test area with no reference capture ligand; at least one test area with no target capture ligand; and wherein the target capture ligands have been placed in a predetermined array.

Preferred embodiments for the above microassay devices include devices where placement of the target capture ligands and reference capture ligand are in a predetermined array, and where the reference capture ligand is placed in least two spaced apart locations in the array. More preferably, the reference capture ligand is placed in at least two corner locations in the array. Most preferably, the reference capture ligand is placed in at least three corner locations in the array.

An assay method, according to the invention, for determining the quantity of a plurality of different target ligands involves a step of selecting the microarray device of the invention that is described above. Another step is placing in at least one of the test areas (i) sample containing target ligands, wherein at least some of the target ligands are captured by target capture ligands, (ii) reference ligand, wherein at least some of the reference ligand is captured by the reference capture ligand, (iii) a reference ligand detector for forming a complex with captured reference ligand, and (iv) target ligand detector for forming a complex with captured target ligands. Another step in this method is detecting the amount of reference ligand detector and target ligand detector that are in complex.

In an embodiment of the above assay method, target capture ligands and reference capture ligands are immobilized in predetermined array on the microarray device, and the reference capture ligands are immobilized onto at least two corner locations in the array.

A method of forming a microarray, according to the invention, for determining the quantity of a plurality of target ligands in a sample, the microarray includes a step of selecting a solid support having a plurality of test areas with substantially the same geometry. Another step is immobilizing a plurality of different target capture ligands and at least one reference capture ligand in a predetermined array onto the test area, the reference capture ligand being selected to capture a reference ligand not normally present in the sample.

Embodiments of the above method of forming the microarray includes forming the microarray where: at least one test area has no reference capture ligand; and where at least one test area has no target capture ligand. Preferably, as in the above method a forming the microarray, the step of immobilizing at least one reference capture ligand, involves the reference capture ligand being immobilized onto at least two corner locations in the array. Most preferably, in the above method of forming the microarray, the step of immobilizing at least one reference capture ligand involves the reference capture ligand being immobilized onto at least three corner locations in the array.

### DRAWINGS

These features, aspects and advantages of the present invention will become better understood with regard to the following description, appended claims and accompanying figures where:
Figure 1 depicts an array within the test area of a microarray, and identifies locations within the array that correspond to internal reference spots and test spots or target ligand spots.
Figure 2 shows a graph comparing the coefficient of variation ("CV") using normalized results of an IL-8 microarray based immunoassay based on the present invention, and adjusted for differences in the printing process and in mixing during the assay.
Figure 3 is a graph, similar to Figure 2, with normalized results of an IL-8 microarray assay of the present invention wherein adjustments are made for different incubating times such as 30, 45, 60 and 75 minutes as the variable.
Figure 4 depicts a predetermined grid or pattern used with the present invention for depositing specific capture oligonucleotides corresponding to a specific target ligand to be captured in an array within a test area.

### DESCRIPTION

The following discussion describes embodiments of the invention and several variations of these embodiments. This discussion should not be construed, however, as limiting the invention to these particular embodiments. Practitioners skilled in the art will recognize numerous other embodiments as well.

### a. Definitions

As used herein, the term "microarray," refers to an array or pattern of discrete capture agents deposited as small spots in a test area on a suitable solid substrate.

As used herein, the term "test area" refers to any surface area corresponding to and immediately surrounding an array within the microarray. For example, "test area" includes surface area within a well in a microplate or the surface area of a glass microscopic slide or the surface area of a bead wherein the spots are deposited as an array.

As used herein, the term "test well" refers to surface area within a well in a microplate or the surface area of a glass microscopic slide wherein the spots are deposited as a distinct array.

As used herein, the term "capture agent" refers to either a "capture ligand" or a capture oligonucleotide capable of hybridizing under appropriate conditions to a complementary oligonucleotide attached to a capture ligand.

As used herein, the term "capture ligand" refers to any biological molecule that captures or binds to a "target ligand."

As used herein, the term "capture oligonucleotide" means an oligonucleotide immobilized or attached to a solid support which can bind a complementary oligonucleotide attached to a capture ligand.

As used herein, the terms "complementary oligonucleotide" or "oligonucleotide complementary to a capture oligonucleotide" refers to a nucleotide sequence attached to a capture ligand that hybridizes to the capture oligonucleotide under conditions suitable for hybridization thereby forming double stranded nucleic acid duplexes.

As used herein, the terms "biological molecule" or "target ligand" or "anti-ligand" encompass any organic molecule, and includes but is not limited to oligonucleotides, nucleic acids, such as DNA and RNA, polypeptides, haptens, and carbohydrates.

As used herein, the terms, "polypeptide" as referred to herein encompasses, and includes but is not limited to proteins and antibodies, and any fragments thereof.

As used herein, the term "haptens" refers to small molecules, such as drugs, hormones, and synthetic compounds including but not limited to compounds associated with the use of therapeutic drugs and drugs of abuse. Examples of haptens associated with drugs of abuse include but are not limited to compounds associated with the metabolism or use of cocaine, morphine, and nicotine. Examples of haptens in terms of therapeutic drugs include but are not limited to compounds associated with the use of tobramycin, phenobarbital, theophylline, digoxin, and gentamicin.

As used herein, the terms "target ligand detector" refers to a labeled anti-ligand that also binds to the target ligand to form a conjugate.

As used herein, the terms "reference ligand" refers to a "biological molecule" which is the same type of biological molecule as the "target ligand," and is used to normalize the microarray assay results for target ligands in the array.

As used herein, the terms "reference ligand detector" refers to a labeled anti-ligand that also binds to the reference ligand to form a conjugate.

As used herein, the terms "orienting" or "orientation" or "aligning" or "alignment" of the microarray refers to positioning the microarray or the detection instrument to align each spot in the array for detection and measurement. The location of the spots within each test area can vary slightly based on the depositing process, and small variation in the location of small spots can affect accuracy.

As used herein, the terms "normalizing the signal" refers to adjusting the signal associated with the target ligands in response to the intensity of the known amount of reference ligand detected, and thereby improving the accuracy of the measurement of target ligands.

### b. The Present Invention

The present invention provides a method: for quantitative measurement of target ligands in the microarray; for adjusting for variation that can occur during the microarray assay to improve the precision of the assay; and that facilitates the orientation of the microarray for more accurate analysis; and permits measurement of captured target ligands using relatively inexpensive and time saving procedures, as defined by the independent claims.

The invention is useful in diagnostic procedures such as assays for a variety of target ligands or biological molecules. The invention can be used, for example, in an assay involving the quantity and presence of cytokines, the presence of a disease state in an organism, the quantity and presence of a therapeutic drug, and detection of nucleic acids resulting from underlying infections.

The invention uses capture agents deposited as discrete spots on a suitable microarray substrate. A suitable microarray substrate can be any surface having the appropriate chemistry wherein a microarray printer or other device can be used to deposit capture agents as microscopic spots on the substrate. Microarrays substrates include but are not limited to the surface of: flat microscope slides; flexible membranes made of nitrocellulose, nylon, and PVDF; and microwell plates made of glass, polyacrylates, polystyrene, polypropylene, and polycarbonate. Microarrays with capture agents already immobilized on their surface can also be commercially purchased from a manufacturer. For example, Pierce Chemical Co, PO Box 117, Rockford, IL 61105 sells 96 well microplates containing wells with a low density array of capture agents (Part numbers 84682 through 84689).

In the invention, the capture agents are deposited as small spots onto the microarray substrate in a designated pattern that corresponds to a location for identifying the presence and quantifying each specific target ligand and reference ligand in the array. Each spot in the pattern corresponds to a capture agent for a particular target ligand or reference ligand or a spot in the array can be empty (*i.e*. no capture agent). The reference ligand provides an internal reference spot or spots in each test area that are subject to the same conditions as the capture agents for the target ligands. Throughout the assay, the internal reference spots in each test area are subject to the same conditions affecting the detection and measurement of the target ligands in the test area, such as variability in depositing the spots, the sample, reagents, temperature, or other variables in the assay procedure.

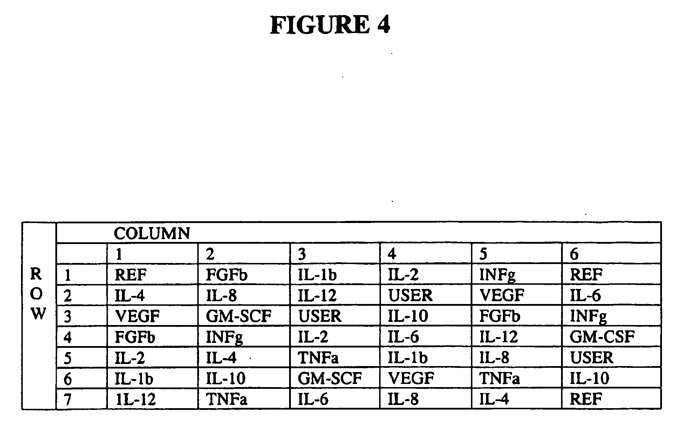

One example of a designated pattern or "Predetermined Grid" corresponding to specific positions in the grid where target ligands and reference ligands would be captured is depicted in Figure 4. In Figure 4, the spots correspond to locations where a target ligand, or a reference ligand are sought to be captured by the assay according to the present invention.

The specific target ligands to be captured in the pattern depicted in Figure 4 are:
IL-1b (Interleuken 1b);
IL-2 (Interleuken 2);
IL-4 (Interleuken 4);
IL-6 (Interleuken 6);
IL-8 (Interleuken 8);
IL-10 (Interleuken 10);
IL-12 (Interleuken 12);
FGFb (fibroblast growth factor basic);
GM-CSF (granulocyte/monocyte colony stimulating factor);
INFg (Interferon gamma);
TNFa (tumor necrosis factor alpha);
VEGF (vascular endothelial growth factor); and
USER (a target ligand chosen by the user or an empty spot (no target ligand)), and is usually a different target ligand than the above target ligands. As one of ordinary skill in the art will understand, there are a variety of different permutations of designated patterns that can be placed in an array in a test area.

The designation REF in Figure 4 is a reference ligand not normally present in the sample subjected to the assay. In the experiment described in Example 1, the reference ligand REF was TNFa, and the USER target ligand site was unused (i.e., an empty spot).

In the present invention, one or more internal reference spots can be deposited onto the test area, and is only limited by the number of spots in the test area. However, as the number of internal reference spots in the test area increases, there is a concomitant decrease in the number of spots available for testing of target ligands.

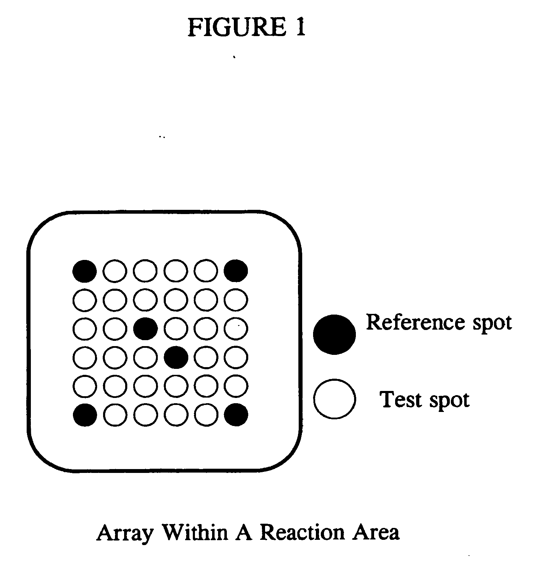

In the invention, internal reference spots are deposited at identical locations within each test area. The internal reference spots can be deposited at any location within the test area. However, the preferred location for internal reference spots is at one or more corners, if corners are present in the shape's configuration. Figure 1 depicts an array within a test area of a microarray, and identifies test spot (or target ligand capture spots) and reference spots (or internal reference ligand capture spots).

In a preferred embodiment of the invention, the capture agents for the reference ligand are deposited in the same three corners of each test area. Locating the capture agents for the reference ligand in the corners permits easier orientation or alignment of the instrument or microarray for more accurate detection of each spot in the array. As references for improving quantitation, the reference spots could be placed anywhere. Placing the spots in 3 of the 4 corners gives the analysis software discrete and reproducible reference points for placing a 'map' used to identify and quantify the remaining spots. With the aid of these discrete and reproducible reference points, software can be designed or modified to automatically locate and map the microarrays following acquisition of the digital photograph with no intervention on the part of the experimenter.

Biological molecules used as capture agents in the method of the present invention are molecules having a reactive group that bind to a suitable microarray substrate during the step of depositing capture agents onto the microarray. Biological molecules with at least one reactive amino, thiol or hydroxyl group can bind to a microarray substrate for use in the present invention. Polypeptides, haptens, and carbohydrates with at least one reactive amino, thiol or hydroxyl group, can be purchased from Sigma, P.O. Box 14508, St. Louis, MO 63178. Oligonucleotides that can bind to a suitable microarray substrate during the step of depositing capture agents onto the microarray include amino derivatized oligonucleotides and oligonucleotides having at least one free thiol group. Amino oligonucleotides can be synthesized on a 3' Amino-Modifier C7 CPG (purchased from Glen Research, 22825 Davis Drive, Sterling, Virginia 20164) following the manufacturer's protocol using a DNA synthesizer ABI 394 (purchased from Applied Biosystems, 850 Lincoln Centre Drive, Foster City, CA 94404). The amino group can be placed at 3' end or at 5' end of oligonucleotide. The 3' amino oligonucleotide is preferably used in the preparation of amino oligonucleotides for the present invention. A method for preparing amino derivatized oligonucleotides is also described in U.S. Patent No. 6,110,669. In addition, oligonucleotides having at least one free thiol group can be synthesized on supports purchased from Glen Research following the manufacturer's protocol.

Spots can be deposited or printed onto a suitable microarray substrate using a variety of different methods. Methods of depositing spots onto a microarray are well known to one of ordinary skill in the arts. *See e.g.,* U.S. Patent No. 6,312,960. For example, and not as a limitation, inkjet technology and piezo electric microjet printing technology have been used to deliver low volumes of solution, and is referred to as "printing" spots on the solid supports. One method of printing biological molecules onto a solid support is described in U.S. Patent No. 6,146,833. Another method of depositing spots is contact printing where a pin is dipped into a solution of the molecule to be printed, and brought into contact with a surface releasing a reproducible volume of liquid. Capillary printing is yet another method of depositing spots in a microarray.

Depending on the type of capture agents deposited as spots onto the microarray, the conditions needed to effectively conduct the assay can vary. However, conditions for employing specific assays are well known to one of ordinary skill in the arts, including but not limited to assays using capture agents such as oligonucleotides, cells, polypeptides, such as antibodies. *See e.g.,* Wang H, Wang H, Zhang W, Fuller GN., Tissue microarrays: applications in neuropathology research, diagnosis, and education., Brain Pathol 2002 Jan;12(1):95-107 (Tissue microarrays); Mousses S, Kallioniemi A, Kauraniemi P, Elkahloun A. Kallioniemi OP., Clinical and functional target validation using tissue and cell microarrays, Curr Opin Chem Biol 2002 Feb;6(1):97-101(Cell microarrays);Wilson DS, Nock S., Functional protein microarrays, Curr Opin Chem Biol 2002 Feb;6(1):81-5 (General protein microarrays): Schweitzer B. Kingsmore SP.. Measuring proteins on microarrays Curr Opin Biotechnol 2002 Feb;13(1):14-9 (Antibody microarrays), and Shoemarker, DD, and Linsley, PS Recent developments in DNA microarrays. Curr Opin Microbiol 5(3):334-7, June 2002 (DNA Microarrays).

When capture oligonucleotides are deposited onto the microarray as the spots, capture ligands attached to complementary oligonucleotides are added at some point in the assay. The capture ligand attached to the complementary oligonucleotide can be antigens, antibodies, binding proteins, haptens, hormone receptors, hormones, lectins, carbohydrates, metabolites, drugs, enzyme substrates, or viral proteins. The attachment of oligonucleotides to antigens, antibodies, binding proteins, haptens, hormone receptors, hormones, lectins, carbohydrates, metabolites, drugs, enzyme substrates, and viral proteins are well known to one of ordinary skill in the arts. *See* U.S. Patent No. 5,648,213. A preferred method of attaching antibodies as conjugates to oligonucleotides is described in Serial No. 10,032592, filed October 24, 2001, entitled "Efficient Synthesis of Protein-Oligonucleotide Conjugates."

At some point in the assay using oligonucleotides, conditions must be suitable to permit complementary oligonucleotides to hybridize to capture oligonucleotides to form nucleic acid duplexes. The conditions conducive to the formation of nucleic acid complexes by oligonucleotides are well known to one of ordinary skill in the arts. (as described, for example, in Sambrook and Russell, Molecular Cloning: A Laboratory Manual, 3rd Edition, Cold Spring Harbor Laboratory Press. New York, or Current Protocols in Molecular Biology, edited by Frederick Ausubel, John Wiley and Sons Publishing. New York, 1987).

Capture and complementary oligonucleotides used in the present invention range from about 15 to about 45 base oligonucleotides. The capture and oligonucleotides preferably each have about 20 to about 30 base oligonucleotides.

Preferred pairs of capture oligonucleotides and complementary oligonucleotides, are described in patent application Serial No. 10/419,020, filed April 18. 2003, Attorney Docket No. 13796, entitled "oligonucleotide Pairs for Multiplexed Binding Arrays." The preferred oligonucleotide pairs hybridize to form nucleic acid duplexes at room temperature, and do not have substantial cross hybridization. This is useful for an assay testing for the presence of multiple target ligands. When testing multiple target ligands according to the present invention, these were the preferred sequences.

In a preferred embodiment of the present invention, the spots deposited onto the microarray are capture oligonucleotides that are different for each target ligand and reference ligand. The capture and complementary oligonucleotides are selected from the pairs identified above.

A variety of different types of detectable labels can be directly attached to the capture or complementary oligonucleotides and used in the present invention. Those detectable labels include but are not limited to fluorophores, radioactive, chemiluminescent, bioluminescent, enzyme, nephelometric, turbidometric, and visible labels. Examples of fluorophores that can be used in the invention include but are not limited to rhodamine 110, rhodal, fluorescein, coumarin, and derivatives of rhodamine 110, rhodal, or fluorescein. Cyanine dyes such as Cy2, Cy3, Cy5, Cy5.5. and Cy7. Examples of radioactive tables that can be used the invention include but are not limited to ³²P, ³³P, ³⁵S, ³H, and ¹²⁵I. Examples of chemiluminescent labels that can be used in the invention include but are not limited to acridinium esters, ruthenium complexes, metal complexes, oxalate ester - peroxide combination. Examples of enzyme labels that can be used in the invention include but are not limited to alkaline phosphatase, horse-radish peroxidase, beta-galactosidase. Examples of visible labels that can be used in the invention include but are not limited to thiopeptolides, anthroquinone dyes, nitro blue tetrazolium, ortho-nitrophenol β-D-galacto-piranoside (ONPG), colloidal gold, and microparticles. The same type of labels, discussed above, can be used on detector ligands. Methods for attachment of detectable labels to oligonucleotides are well known to one of ordinary skill in the arts. For example, such methods are described in Yang and Millar, Methods in Enzymology, Vol. 278, pages 417-444, 1997.

Measurement and detection of the labels is dependent on the type of label used in the assay, and would be based on the manufacturer or other protocols which are well known to one of ordinary skill in the art. *See,* for example, U.S. Patent 5,316,906, which describes the use of enzyme substrates that form fluorescent precipitates. When using fluorophores as the labels in the assay data can be collected in the form of a digital photograph of the entire microarray. The reference spots and the target ligand specific spots can be detected simultaneously in the digital photograph.

Upon or after detection of the labels, an algorithm is used with the collected data from the reference spots to normalize or scale the data pertaining to the other spots in the microarray. While many normalization algorithms are possible, one algorithm was used to generate the date shown in the tables and examples of the invention; namely, the target ligand specific signal (spot) was divided by the mean of the results from the number of reference spots.

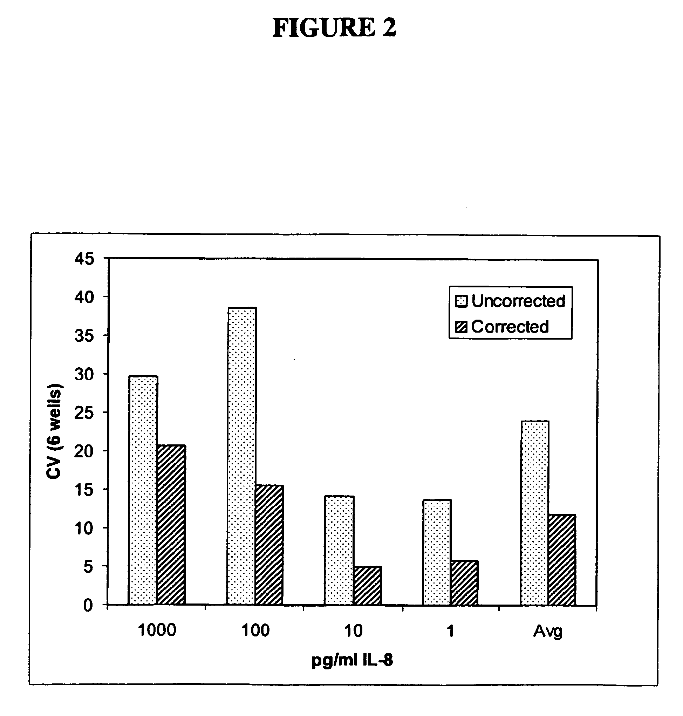

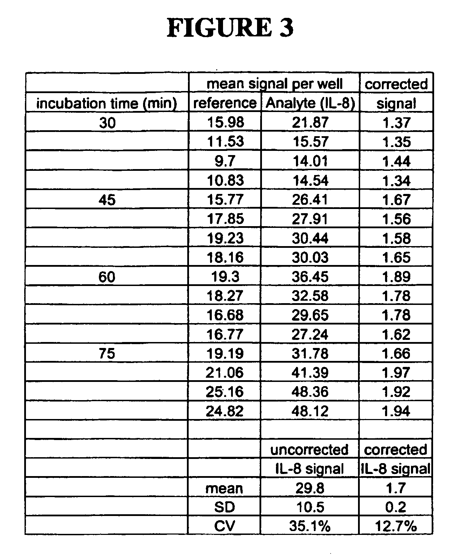

Because quantitation of the reference spots is based on a known quantity of reference ligand subjected to the same conditions as other spots on the same microarray, the results from unknown concentrations of target ligands can be scaled, and improve their reproducibility from well to well. Figure 2 is a graph using normalized results of an IL-8 microarray of the present invention, and adjustments are made for the printing process and mixing in the assay Figure 2 shows the improvement in precision (*i.e.* reduction of CV) of signal intensity produced by this method at 1000. 100, 10, and 1 pg/mL IL-8 as printing processes prior to and mixing during the assay procedure were varied. Figure 3 is a graph, similar to Figure 2, with normalized results of an IL-8 microarray assay of the present invention wherein adjustments are made for different incubating times such as 30, 45, 60 and 75 minutes as the variable

The present invention provides a system that uses internal reference spots to reduce microarray variation. The present invention's system includes microassay devices, assay methods, and methods of forming microassays.

A microarray device for determining the quantity of a plurality of target ligands in a sample, according to the present invention, comprises a solid support having a plurality of test areas; a plurality of different target capture ligands immobilized onto the test areas; and at least one reference capture ligand immobilized onto the test areas, the reference capture ligand being selected to capture a reference ligand not normally present in the sample.

Another microarray device for determining the quantity of a plurality of target ligands in a sample, according to the present invention, comprises a solid support having a plurality of test areas with substantially the same geometry on the solid support; a plurality of different target capture ligand immobilized onto the test areas; and at least one reference capture ligand immobilized onto the test areas, the reference capture ligand being selected to capture a reference ligand not normally present in the sample, the reference capture ligands being in substantially the same location in each test area.

Embodiments of the above microassay devices include devices that have or more of the following: at least one test area with no reference capture ligand,; at least one test area with no target capture ligand; and wherein the target capture ligands have been placed in a predetermined array.

Preferred embodiments for the above microassay devices include devices where placement of the target capture ligands and reference capture ligand are in a predetermined array, and where the reference capture ligand is placed in least two spaced apart locations in the array. More preferably, the reference capture ligand is placed in at least two corner locations in the array. Most preferably, the reference capture ligand placed in at least three corner locations in the array.

An assay method, according to the invention, for determining the quantity of a plurality of different target ligands involves a step of selecting the microarray device of the invention that ie described above. Another step is placing in at least one of the test areas (i) sample containing target ligands, wherein at least some of the target ligands are captured by target capture ligands, (ii) reference ligand, wherein at least some of the reference ligand is captured by the reference capture ligand, (iii) a reference ligand detector for forming a complex with captured reference ligand, and (iv) target ligand detector for forming a complex with captured target ligands. Another step in this method is detecting the amount of reference ligand detector and target ligand detector that are in complex.

In an embodiment of the above assay method, target capture ligands and reference capture ligands are immobilizing in a predetermined array on the microarray device, and the reference capture ligands are immobilized onto at least two corner locations in the array.

A method of forming a microarray, according to the invention, for determining the quantity of a plurality of target ligands in a sample, the microarray includes a step of selecting a solid support having a plurality of test areas with substantially the same geometry. Another step is immobilizing a plurality of different target capture ligands and at least one reference capture ligand in a predetermined array onto the test area, the reference capture ligand being selected to capture a reference ligand not normally present in the sample.

Embodiments of the above method of forming the microarray includes forming the microarray where: at least one test area has no reference capture ligand,; and where at least one test area has no target capture ligand. Preferably, in the above method of forming the microarray, the step of immobilizing at least one reference capture ligand, involves the reference capture ligand being immobilized onto at least two corner locations in the array. Most preferably, in the above method of forming the microarray, the step of immobilizing at least one reference capture ligand involves the reference capture ligand being immobilized onto at least three corner location in the array.

The present invention is not limited to the preferred embodiments described in this section. The embodiments are merely exemplary, and one skilled in the art will recognize that many others are possible in accordance with this invention. Having now generally described the invention, the same will be more readily understood through references to the following examples, which are provide by way of illustration, and are not intended to be limiting of the present invention, unless so specified.

### EXAMPLE 1

### PREPARATION AND ANALYSIS OF A MICROARRAY ASSAY USING A MICROPLATE ACCORDING TO THE PRESENT INVENTION

In this example, an A²(A² is an abbreviation for an array within an array) plate was the microtiter plate used as the microarray substrate. The A² plate is not yet commercially available. The A² plate was a polypropylene plate that consisted of 96 test wells, and was manufactured by Beckman Coulter. A pretreatment procedure was used for the surface of the A² microtiter plate to give is a chemistry that reacts with the amine groups on the oligonucleotide, and that procedure was described in patent application Serial No. 10/033,308, filed October 24, 2001, entitled Immobilizing Biological Molecules.

42 spots of a solution containing capture oligonucleotides were primed as a microarray into each of the 96 test wells using a commercial non-contact array printer PixSys 4200 (Cartesian Technologies, Inc. 17851 Sky Park Circle Suite C, Irvine, CA, 92614).

The printing solution that was used in this example consisted of 20µM capture amino-oligonucleotide generated by using a 10nL drop of 20µM solution to an alkaline buffer of 50mM carbonate + 4% w/v sodium sulfate. The capture oligonucleotides were printed in a low dust, high humidity environment onto the pretreated surface of the A² plate. Once deposited on the surface, the spots were incubated overnight (approximately 16 hours) before being washed off. Residual activation chemistry on the plate was then deactivated by reacting the plate with a large excess of solution of amine containing molecule. After rinsing and drying the plate was then ready for use.

The 42 spots of capture oligonucleotides in each test well corresponded to the following:
a. 12 different target ligands that were to be detected and quantified at each spot;
b. 1 set of reference spots; and
c. 1 set of spots for target ligands other than the 12 target ligands specified above, corresponding to a user selected target ligand or a user selected spot with no capture target ligand (USER) in the grid shown in Figure 4.

Each of the above capture oligonucleotides were deposited in triplicate into predetermined locations in the array. Therefore, 3 x (times) 14 spots equals the total of 42 spots in the array. The three reference spots were deposited in the corners, and provided information about orientation and position of the microarray.

The predetermined grid (positions) for printing the capture oligonucleotides corresponding to specific target ligands in the arrays used in Example 1 are shown in Figure 4 previously identified in the description. In Figure 4, REF refers to the reference ligand, and USER refers to a user selected target ligand or empty location as described above. The reference ligand that was used for Example 1 was TNFa and the USER spot had no capture target ligand.

In this example, a different capture oligonucleotide was deposited for each of the 13 different sets of spots, and no capture oligonucleotide for the USER spot. The capture oligonucleotide was selected from the previously identified preferred sequence pairs, and printed onto the microarray substrate. The (other) complimentarynucleotides of each sequence pair, was conjugated to an antibody corresponding to its target ligand using the method described in Serial No. 10,032592, filed on October 24, 2001, and entitled Efficient Synthesis of Protein-Oligonucletode Conjugates. Under appropriate conditions, the sequence pairs hybridized to form nucleic acid duplexes.

### Hybridization Buffer:

Superblock buffer was prepared according to manufacturer's directions (Pierce Biotechnology, PO Box 117. Rockford, IL, 61105). The Superblock buffer was mixed with formamide as follows Superblock:formamide = 3:1. This buffer was used immediately upon formulation.

### Method:

a) Prepared a mixture of the desired monoclonal-oligonucleotide conjugates (1.4µg/mL of each) in Hybridization Buffer 2. All 12 analyte specific conjugates plus the ovalbumin reference conjugate were used in this example.
b) The wells were rinsed briefly with wash buffer, then 70µL of the antibody-oligonucleotide conjugate mixture was added to each well.
c) The plate was covered with a polystyrene lid and mixed for 1 hour at room temperature.
d) The plate was washed by adding 200µL of wash buffer to each well, and mixing for 1 minute. The bulk of the wash buffer was removed by quickly inverting the plate over a sink. The plate was tapped onto several layers of paper towels to remove residual buffer. This process was repeated twice for a total of three times.
e) A 1:10 dilution of SuperBlock in TBS was prepared for use in the next step.
f) 35µL of the diluted SuperBlock was added to each well, followed by 35µL of sample added to each well.
g) Incubated as described in step (c above.
h) Washed as in described in step (d above.
f) A mixture of the desired polyclonal antibody-biotin conjugates (0.25µg/mL of each) in diluted SuperBlock buffer were prepared.
j) 70µL of the polyclonal antibody-biotin conjugate mixture was added to each well.
k) Incubated as described in step (c above.
l) Washed as in described in step (d above.
m) Prepared a dilution of streptavidin-PBXL1 (Martek Biosciences Corporation, Columbia, MD) to 6.7µg/mL (1:150 dilution of the 1mg/mL stock solution) in diluted SuperBlock buffer.
n) 70µL of the diluted streptavidin-PBXL1 was added to each well.
o) Incubated as described in step (c above.
p) Washed as in described in step (d above.
q) 200µL of wash buffer was added to each well and the plate stored in the dark at 4°C until imaged. The wash buffer from the wells was not removed during imaging in order to preserve PBXL fluorescence.

The instrument used to measure the fluorescence was a Beckman Coulter breadboard version of an A² plate reader that is not yet commercially available. This instrument consisted of an automated stage that held the plate, a CCD camera (Roper Coolsnap CF), and a white light source. The plates were illuminated using, a 550 nm excitation light source and visualized with the CCD camera mounted with a 675 nm emission filter to detect the PBXL1 labels for the bound antigens in the array. The antigens correlated to the positions identified on the grid shown in Figure 4. A digital photograph was taken of the array using the Roper Coolsnap CF.

The resulting images were quantified using the commercial software Imagene noted above according to the manufacturer's directions. The formula used for normalization of the results was the target ligand specific signal (spot) divided by the mean of the results from the number of reference spots.

### EXAMPLE 2

### PREPARATION AND ANALYSIS OF A MICROARRAY ASSAY USING A MICROPLATE AND BIOTIN MOIETIES ACCORDING TO THE PRESENT INVENTION

In this example, an A² plate was the microtiter plate used as the microarray substrate. The A² plate is not yet commercially available. The A² plate was a polypropylene plate that consisted of 96 test wells, and was manufactured by Beckman Coulter. A pretreatment procedure was used for the surface of the A² microtiter plate to give it a chemistry that reacts with the amine groups on the oligonucleotide, and that procedure was described in patent application Serial No. 10/033,308, filed October 24, 2001, entitled Immobilizing Biological Molecules.

42 spots of a solution containing capture oligonucleotides were printed as a microarray into each of the 96 test wells using a commercial non-contact array printer PixSys 4200 (Cartesian Technologies, Inc. 17851 Sky Park Circle Suite C, Irvine, CA, 92614).

The printing solution that was used in this example consisted of 20µM capture amino-oligonucleotide generated by using a 12nL drop of 20µM solution in an alkaline buffer. The capture oligonucleotides were printed in a low dust, high humidity environment onto the pretreated surface of the A² plate. Once deposited on the surface, the spots were incubated overnight (approximately 16 hours) before being washed off. Residual activation chemistry on the plate was then deactivated by reacting the plate with a large excess of solution of amine containing molecule. After rinsing and drying the plate was then ready for use.

The 42 spots of capture oligonucleotides in each test well corresponded to the following:
a. 12 different target ligands that were to be detected and quantified at each spot:
b. 1 set of reference spots; and
c. 1 set of spots for target ligands other than the 12 target ligands specified above, corresponding to a user selected target ligand or a user selected spot with no capture target ligand (USER) in the grid shown in Figure 4.

Each of the above capture oligonucleotides were deposited in triplicate into predetermined locations in the array. Therefore, 3x (times) 14 spots equals the total of 42 spots in the array. The three reference spots were deposited in the corners, and provided information about orientation and position of the microarray.

The predetermined grid (positions) for printing the capture oligonucleotides corresponding to specific target ligands in the arrays used in Example 1 are shown in Figure 4 previously identified in the description. In Figure 4, REF refers to the reference ligand, and USER refers to a user selected target ligand or empty location as described above. The reference ligand that was used for Example 2 was TNFa and the USER spot bad no capture target ligand.

In this example, a different capture oligonucleotide was deposited for each of the 14 different sets of spots, including the reference spots. Capture oligonucleotides were selected from the previously identified preferred sequence pairs, and printed onto the microarray substrate. The complimentary oligonucleotides of each sequence pair were conjugated to an antibody corresponding to its target ligand using the method described in Serial No. 10/032,592, filed on October 24, 2001, and entitled Efficient Synthesis of Protein-Oligonucleotide Conjugates, with the exception of the oligonucleotide complimentary to the sequence attached to the microarray's reference spots. The sequence complementary to the reference spots was synthesized with a biotin moiety in the 3' position. Under appropriate conditions, the sequence pairs hybridized to form nucleic acid duplexes.

### Hybridization Buffer:

Superblock buffer was prepared according to manufacturer's directions (Pierce Biotechnology, PO Box 117. Rockford, IL, 61105). The Superblock buffer was mixed with formamide as follows Superblock:formamide = 3:1. This buffer was used immediately upon formulation.

### Method:

a) Prepared a mixture of the desired monoclonal-oligonucleotide conjugates (1.4µg/mL of each) and the biotinylated reference oligonucleotide at 20nM in Hybridization Buffer.
b) The wells were rinsed briefly with wash buffer, then 70µL of the antibody-oligonucleotide conjugate/reference biotinylated oligonucelotide mixture was added to each well.
c) The plate was covered with a polystyrene lid and mixed for 1 hour at room temperature.
d) The plate was washed by adding 200µL of wash buffer to each well, and mixing for 1 minute. The bulk of the wash buffer was removed by quickly inverting the plate over a sink. The plate was tapped onto several layers of paper towels to remove residual buffer. This process was repeated twice for a total of three times.
e) A 1:10 dilution of SuperBlock in TBS was prepared for use in the next step.
f) 35µL of the diluted SuperBlock was added to each well, followed by 35µL of sample added 10 each well.
g) Incubated as described in step (c above.
h) Washed as in described in step (c above.
i) A mixture of the desired polyclonal antibody-biotin conjugates (0.25µL/mL of each) in diluted SuperBlock buffer were prepared.
j) 70µL of the polyclonal antibody-biotin conjugate mixture was added to each well.
k) Incubated as described in step (c above.
l) Washed as in described step (d above.
m) Prepared a dilution of streptavidin-PBXL1 (Martek Biosciences Corporation, Columbia, MD) to 6.7µg/mL (1:150 dilution of the 1mg/mL stock solution) in diluted SuperBlock buffer.
n) 70µL of the diluted streptavidin-PBXL1 was added to each well.
o) Incubated as described in step (c above.
p) Washed as in described in step (d above.
q) 200µL of wash buffer was added to each well and the plate stored in the dark at 4°C until imaged. The wash buffer from the wells was not removed during imaging in order to preserve PBXL fluorescence.

The instrument used to measure the fluorescence was a Beckman Coulter breadboard version of an A² plate reader that is not yet commercially available. This instrument consisted of an automated stage that held the plate, a CCD camera (Roper Coolsnap CF), and a white light source. The plates were illuminated using, a 550 nm excitation light source and visualized with the CCD camera mounted with a 675 nm emission filter to detect the PBXL1 labels for the bound antigens in the array. The antigens correlated to the positions identified on the grid shown in Figure 4. A digital photograph was taken of the array using the Roper Coolsnap CF.

The resulting images were quantified using the commercial software Imagene noted above according to the manufacturer's directions. The formula used for normalization of the results was the target ligand specific signal (spot) divided by the mean of the results from the number of reference spots.

### EXAMPLE 3

### PROSPECTIVE EXAMPLE USING INVENTION WITH AN ANTIBODY BASED ASSAY

A microarray consisting of different specific antibodies can be produced using techniques, chemistries, and surfaces currently used for protein microarrays. These can subsequently be used for quantitation of specific antigenes by either competitive or non-competitive assays. Competitive assays are performed by permitting antigens in the sample to compete with limiting amounts of labeled (frequently biotinylated) antigen or antigen analog for binding to the specific antibody. The amount of labeled antigen that is bound to the specific antibody is inversely related to the concentration of antigen in the sample. Reference spots consisting of antibody to an analyte not present in the sample could be used in conjunction with a carefully controlled concentration of labeled reference antigen to provide both an internal reference for normalization of other assay results within the microarray and as a set of internal reference points for automated data acquisition. Non-competitive assays can be performed by permitting antigens in the sample to bind to the immobilized specific antibodies. Excess material can be removed by washing, after which a second antibody or set of antibodies specific for different sites on the same antigenic molecule can be added and allowed to bind. These can be directly labeled with a fluorescent or enzymatic 'tag' molecule or indirectly labeled with a moiety (such as biotin) that is subsequently labeled in a later step. In either case the amount of the second antibody bound to the site is directly proportional to the concentration of antigen in the sample. Reference spots consisting of: (1) antibody to an analyte not present in the sample can be used in conjunction with carefully controlled concentrations of reference antigen, and (2) a second reference antigen-specific antibody to a different site on the reference antigen to provide both an internal reference for normalization of other assay results within the microarray and as a set of internal reference points for automated data acquisition.

### EXAMPLE 4

### PROSPECTIVE EXAMPLE USING INVENTION WITH A CELL BASED ASSAY

Cell or tissue microarrays may also be assembled by distributing different cell types or thin sections taken from specific tissues on a solid surface. The contents of theses may then be studied for genetic content by in situ hybridization with DNA or RNA probes or for specific proteins by immunostaining with labeled antibodies. Use of replicate microarrays allows characterization of the same specimens by a variety of different probes and/or antibodies. A set of reference spots consisting of a standard cell or tissue preparation would permit relative quantitation of the targeted analytes in addition to providing orientation landmarks for quantitation software.

Although the present invention has been described in considerable detail with reference to certain preferred versions thereof, other versions are possible.

## Claims

1. A microarray device for detecting and quantifying a plurality of target ligands in a sample, the microarray comprising:
a) a solid support having a plurality of test areas;
b) a plurality of different target capture ligands immobilized onto the test areas; and
c) at least one reference capture ligand immobilized onto the test areas at a location where no target capture ligands are present, the reference capture ligand being selected to capture a reference ligand not normally present in the sample, the reference capture ligands being in substantially the same location in each test area;
wherein the reference capture ligand is usable to orient the microarray device and quantify the target ligands in the sample, and spots corresponding to a capture agent for a particular target ligand or reference ligand or spots containing no capture agent are deposited onto the microcarrier substrate in a designated pattern that corresponds to the location for identifying the presence and quantifying each specific target ligand in the array,
wherein the target capture ligands and reference capture ligand are placed in a predetermined array, and the reference capture ligand is in at least three corner locations in the array.

2. The microarray device of claim 1 wherein the plurality of test areas are a plurality of test wells.

3. The microarray device of claim 1 wherein the plurality of test areas are a plurality of test wells having substantially the same geometry.

4. The microarray device of claim 3 wherein at least one test well has no reference capture ligand.

5. The microarray device of claim 3 or 4 wherein at least one test well contains no target capture ligand.

6. An assay method for determining the quantity of a plurality of different target ligands in a sample comprising the steps of:
a) selecting the microarray of claim 2 or 3;
b) placing in at least one of the test wells (i) a sample containing target ligands, wherein at least some of the target ligands are captured by target capture ligands, (ii) a reference ligand not normally present in the sample, wherein at least some of the reference ligand is captured by the reference capture ligand, (iii) a reference ligand detector for forming a complex with captured reference ligand, and (iv) a target ligand detector for forming a complex with captured target ligands;
c) detecting the amount of reference ligand detector and target ligand detector that are in complex;
d) orienting the microarray with reference to the reference ligand detected; and
e) the reference spots containing the reference ligand are references for improving quantitation and the results from unknown concentrations of target ligands can be scaled because quantitation of the reference spots is based on a known quantity of reference ligand,
wherein the step of placing in at least one of the test wells, further comprises immobilizing the target capture ligands and reference capture ligand in a predetermined array; and immobilizing the reference capture ligand onto at least three corner locations in the array.

7. A method of forming a microarray for detecting and quantifying a plurality of target ligands in a sample, the method comprising the steps of:
a) selecting a solid support having a plurality of test wells with substantially the same geometry; and
b) immobilizing a plurality of different target capture ligands and at least one reference capture ligand in a predetermined array onto the test well, the reference capture ligand being selected to capture a reference ligand not normally present in the sample, and the reference capture ligand being immobilized at a location where no target capture ligands are present, and wherein the reference capture ligand is usable to orient the microarray device and quantify the target ligands in the sample, and spots corresponding to a capture agent for a particular target ligand or reference ligand or spots containing no capture agent are deposited onto the microcarrier substrate in a designated pattern that corresponds to the location for identifying the presence and quantifying each specific target ligand,
wherein the step of immobilizing at least one reference capture ligand further comprises immobilizing the reference capture ligand onto at least three corner locations in the array.

8. The method of forming the microarray of claim 7 wherein at least one well has no reference capture ligand.

9. The method of forming the microarray of claim 7 or 8 wherein at least one well contains no target capture ligand.

## Patentansprüche

1. Mikroarray-Vorrichtung zum Nachweisen und Quantifizieren einer Vielzahl von Zielliganden in einer Probe, wobei das Mikroarray
a) einen festen Träger mit einer Vielzahl von Testbereichen;
b) eine Vielzahl verschiedener Zieleinfangliganden, welche auf den Testbereichen immobilisiert sind, und
c) mindestens einen Referenzeinfangliganden umfasst, welcher auf den Testbereichen an einer Stelle immobilisiert ist, an der keine Zieleinfangliganden vorliegen,
wobei der Referenzeinfangligand ausgewählt ist, um einen normalerweise nicht in der Probe vorliegenden Referenzliganden einzufangen, wobei sich die Referenzeinfangliganden in jedem Testbereich im Wesentlichen an der gleichen Stelle befinden,
wobei der Referenzeinfangligand verwendbar ist, um die Mikroarray-Vorrichtung auszurichten und die Zielliganden in der Probe zu quantifizieren, und wobei Flecken, die einem Einfangmittel für einen speziellen Zielliganden oder Referenzliganden entsprechen, oder Flecken, die kein Einfangmittel enthalten, auf dem Mikroträger-Substrat in einem bestimmten Muster abgelagert sind, welches der Stelle zum Identifizieren der Gegenwart und Quantifizieren jedes spezifischen Zielliganden in dem Array entspricht,
wobei die Zieleinfangliganden und der Referenzeinfangligand in einem vorbestimmten Array angeordnet sind und der Referenzeinfangligand an mindestens drei Eckstellen in dem Array vorliegt.

2. Mikroarray-Vorrichtung nach Anspruch 1, wobei die Vielzahl von Testbereichen eine Vielzahl von Testvertiefungen ist.

3. Mikroarray-Vorrichtung nach Anspruch 1, wobei die Vielzahl von Testbereichen eine Vielzahl von Testvertiefungen mit im Wesentlichen der gleichen Geometrie ist.

4. Mikroarray-Vorrichtung nach Anspruch 3, wobei mindestens eine Testvertiefung keinen Referenzeinfangliganden aufweist.

5. Mikroarray-Vorrichtung nach Anspruch 3 oder 4, wobei mindestens eine Testvertiefung keinen Zieleinfangliganden aufweist.

6. Assay-Verfahren zum Bestimmen der Menge einer Vielzahl von verschiedenen Zielliganden in einer Probe, umfassend die Schritte:
a) das Auswählen des Mikroarrays von Anspruch 2 oder 3,
b) das Anordnen in mindestens einer der Testvertiefungen (i) einer Zielliganden enthaltenden Probe, wobei mindestens einige der Zielliganden durch Zieleinfangliganden eingefangen werden, (ii) eines normalerweise nicht in der Probe vorliegenden Referenzliganden, wobei mindestens einiges des Referenzligandens durch den Referenzeinfangliganden eingefangen wird, (iii) eines Referenzliganden-Detektors zum Bilden eines Komplexes mit eingefangenem Referenzliganden, und (iv) eines Zielliganden-Detektors zum Bilden eines Komplexes mit eingefangenen Zielliganden,
c) das Nachweisen der Menge von Referenzliganden-Detektor und Zielliganden-Detektor, welche im Komplex vorliegen,
d) das Ausrichten des Mikroarrays mit Bezug zu dem nachgewiesenen Referenzliganden, und
e) wobei die Referenzflecken, welche den Referenzliganden enthalten, Referenzen zum Verbessern der Quantifizierung sind und die Ergebnisse von unbekannten Konzentrationen von Zielliganden skaliert werden können, da die Quantifizierung der Referenzflecken auf einer bekannten Menge von Referenzligand basiert,
wobei der Schritt des Anordnens in mindestens einer der Testvertiefungen weiter das Immobilisieren der Zieleinfangliganden und Referenzeinfangliganden in einem vorbestimmten Array, und das Immobilisieren des Referenzeinfangliganden an mindestens drei Eckstellen in dem Array umfasst.

7. Verfahren zum Bilden eines Mikroarrays zum Nachweisen und Quantifizieren einer Vielzahl von Zielliganden in einer Probe, wobei das Verfahren die Schritte
a) des Auswählens eines festen Trägers mit einer Vielzahl von Testvertiefungen mit im Wesentlichen der gleichen Geometrie, und
b) des Immobilisierens einer Vielzahl verschiedener Zieleinfangliganden und mindestens eines Referenzeinfangliganden in einem vorbestimmten Array auf der Testvertiefung umfasst,
wobei der Referenzeinfangligand ausgewählt ist, um einen normalerweise nicht in der Probe vorliegenden Referenzliganden einzufangen, und der Referenzeinfangligand an einer Stelle immobilisiert ist, an der keine Zieleinfangliganden vorliegen, und
wobei der Referenzeinfangligand verwendbar ist, um die Mikroarray-Vorrichtung auszurichten und die Zielliganden in der Probe zu quantifizieren, und wobei Flecken, die einem Einfangmittel für einen speziellen Zielliganden oder Referenzliganden entsprechen, oder Flecken, die kein Einfangmittel enthalten, auf dem Mikroträger-Substrat in einem bestimmten Muster abgelagert sind, welches der Stelle zum Identifizieren der Gegenwart und Quantifizieren jedes spezifischen Zielliganden entspricht,
wobei der Schritt des Immobilisierens von mindestens einem Referenzeinfangliganden weiter das Immobilisieren der Referenzeinfangliganden an mindestens drei Eckstellen in dem Array umfasst.

8. Verfahren zum Bilden des Mikroarrays nach Anspruch 7, wobei mindestens eine Vertiefung keinen Referenzeinfangliganden aufweist.

9. Verfahren zum Bilden des Mikroarrays nach Anspruch 7 oder 8, wobei mindestens eine Vertiefung keinen Zieleinfangliganden aufweist.

## Revendications

1. Dispositif à microréseau destiné à la détection et la quantification d'une pluralité de ligands cibles dans un échantillon, le microréseau comprenant :
a) un support solide possédant une pluralité de zones de test ;
b) une pluralité de différents ligands de capture de cible immobilisés sur les zones de test ; et
c) au moins un ligand de capture de référence immobilisé sur les zones de test à un emplacement où aucun ligand de capture de cible n'est présent, le ligand de capture de référence étant choisi pour capturer un ligand de référence qui n'est normalement pas présent dans l'échantillon, les ligands de capture de référence étant essentiellement au même emplacement dans chaque zone de test ;
dans lequel le ligand de capture de référence est utilisable pour orienter le dispositif à microréseau et quantifier les ligands cibles dans l'échantillon, et des points correspondant à un agent de capture pour un ligand cible particulier ou un ligand de référence ou des points ne contenant pas d'agent de capture sont déposés sur le substrat du microsupport selon un motif désigné qui correspond à l'emplacement destiné à identifier la présence et à quantifier chaque ligand cible spécifique dans le réseau,
dans laquelle les ligands de capture de cible et le ligand de capture de référence sont placés dans un réseau prédéterminé, et le ligand de capture de référence se trouve dans au moins trois emplacements situés dans un coin sur le réseau.

2. Dispositif à microréseau selon la revendication 1, dans lequel la pluralité de zones de test est une pluralité de puits de test.

3. Dispositif à microréseau selon la revendication 1, dans lequel la pluralité de zones de test est une pluralité de puits de test possédant essentiellement la même géométrie.

4. Dispositif à microréseau selon la revendication 3, dans lequel au moins un puits de test ne contient pas de ligand de capture de référence.

5. Dispositif à microréseau selon la revendication 3 ou 4, dans lequel au moins un puits de test ne contient pas de ligand de capture de cible.

6. Procédé de dosage pour déterminer la quantité d'une pluralité de ligands cibles différents dans un échantillon, comprenant les étapes suivantes :
a) choisir le microréseau selon la revendication 2 ou 3 ;
b) placer dans au moins un des puits de test (i) un échantillon contenant des ligands cibles, dans lequel au moins une partie des ligands cibles est capturée par des ligands de capture de cible, (ii) un ligand de référence qui n'est normalement pas présent dans l'échantillon, dans lequel au moins une partie du ligand de référence est capturée par le ligand de capture de référence, (iii) un détecteur de ligand de référence pour former un complexe avec le ligand de référence capturé, et (iv) un détecteur de ligand cible pour former un complexe avec les ligands cibles capturés ;
c) détecter la quantité de détecteur de ligand de référence et de détecteur de ligand cible se trouvant dans le complexe ;
d) orienter le microréseau en se référant au ligand de référence détecté ; et
e) les points de référence contenant le ligand de référence sont des références destinées à améliorer la quantification et les résultats issus des concentrations inconnues des ligands cibles peuvent être ajustés car la quantification des points de référence est basée sur une quantité connue de ligand de référence,
dans lequel l'étape de placement dans au moins un des puits de test comprend en outre l'immobilisation des ligands de capture de cible et du ligand de capture de référence dans un réseau prédéterminé ; et l'immobilisation du ligand de capture de référence sur au moins trois emplacements situés dans un coin sur le réseau.

7. Procédé de formation d'un microréseau pour détecter et quantifier une pluralité de ligands cibles dans un échantillon, le procédé comprenant les étapes suivantes :
a) choisir un support solide possédant une pluralité de puits de test ayant essentiellement la même géométrie ; et
b) immobiliser une pluralité de différents ligands de capture de cible et au moins un ligand de capture de référence dans un réseau prédéterminé sur le puits de test, le ligand de capture de référence étant choisi pour capturer un ligand de référence qui n'est normalement pas présent dans l'échantillon, le ligand de capture de référence étant immobilisé à un emplacement où aucun ligand de capture de cible n'est présent, et dans lequel le ligand de capture de référence est utilisable pour orienter le dispositif à microréseau et quantifier les ligands cibles dans l'échantillon, et des points correspondant à un agent de capture pour un ligand cible particulier ou un ligand de référence ou des points ne contenant pas d'agent de capture sont déposés sur le substrat du microréseau selon un motif désigné qui correspond à l'emplacement destiné à identifier la présence et à quantifier chaque ligand cible,
dans laquelle l'étape consistant à immobiliser au moins un ligand de capture de référence comprend en outre l'immobilisation du ligand de capture de référence sur au moins trois emplacements situés dans un coin sur le réseau.

8. Procédé de formation d'un microréseau selon la revendication 7, dans lequel au moins un puits ne contient pas de ligand de capture de référence.

9. Procédé de formation du microréseau selon la revendication 7 ou 8, dans lequel au moins un puits ne contient pas de ligand de capture de cible.
